# EUROPEAN PATENT SPECIFICATION

(11) **EP 4 051 658 B1**
(45) Date of publication and mention of the grant of the patent: **04.09.2024**
(21) Application number: 20816324.6
(22) Date of filing: 30.10.2020
(51) Int. Cl.: C07C 51/265, C07C 51/43, C07C 51/47, C07C 63/26

(54) **SOLID-LIQUID SEPARATION PROCESSES**
VERFAHREN ZUR FEST-FLÜSSIG-TRENNUNG
PROCÉDÉS DE SÉPARATION SOLIDE-LIQUIDE

(30) Priority: 31.10.2019 US 201962928407 P
(43) Date of publication of application: 07.09.2022
(73) Proprietor: INEOS US CHEMICALS COMPANY, Naperville, IL 60563 (US)
(72) Inventor: KEYES, Timothy, H., Naperville, IL 60563 (US)
(74) Representative: King, Alex
(86) International application number: PCT/US2020/058348
(87) International publication number: WO 2021/087352

(56) References cited:
- WO-A1-2012/107733
- WO-A1-2018/125459
- WO-A1-2019/019012

## Description

### RELATED APPLICATIONS

### FIELD

The present disclosure relates generally to solid/liquid separations in processes for manufacturing aromatic carboxylic acids.

### TECHNICAL BACKGROUND

In processes in the chemical, food, and pharmaceutical industries, various separation techniques are used to isolate one material from another. Common techniques for separating solid materials from a liquid include vacuum or pressure filtration, drying, centrifugation, sedimentation and clarification. When a very pure solid product is required, separation may occur in multiple stages and may be combined with washing steps. For example, a solid recovered from one of the techniques noted above may be washed or reslurried with additional liquids in order to remove impurities before being subjected to another solid/liquid separation technique to recover a final, more pure product.

Multiple-stage separation techniques may result in higher purities of solid products, but may require substantially more investment in equipment. One highly successful method to reduce capital expenditures in a multi-stage separation is through the use of a rotary pressure filter apparatus. Rotary pressure filter apparatuses have been designed to perform more than one of the steps of a multiple-stage separation technique in a single piece of equipment by progressing the material being processed through separate work zones. For example, known rotary pressure filter apparatuses perform a filtration in a filter or feed zone to form a filter cake, followed by a washing of the filter cake in one or more wash zones. The washed filter cake may be dried in a drying zone before leaving the rotary pressure filter. Rotary pressure filter apparatus are generally known in the art and are disclosed, for example, in U.S. Pat. Nos. 2,741,369, 7,807,060, and U.S. Patent Application Publication No. 2005/0051473. A process for manufacturing an aromatic carboxylic acid is disclosed in WO 2019/019012 A1.

There remains a need to improve separation processes for aromatic carboxylic acid reaction effluents that utilize rotary pressure filter apparatus.

### SUMMARY

The scope of the present disclosure is not affected to any degree by the statements within the summary.

According to one aspect of the disclosure, a process for manufacturing an aromatic carboxylic acid includes
oxidizing in a reactor zone a feedstock comprising a substituted aromatic hydrocarbon in the presence of an oxidation catalyst and monocarboxylic acid solvent under reaction conditions suitable to form crude aromatic carboxylic acid;
cooling the reactor zone effluent to form a solid/liquid mixture comprising a solid crude aromatic carboxylic acid, a monocarboxylic acid solvent, and minor amounts of the oxidation catalyst;
filtering the solid/liquid mixture in a feed zone of a rotary filter (e.g., a rotary pressure filter), the feed zone having at least two filter zones to form
   a first feed filtrate comprising monocarboxylic acid solvent and solids, and
   a second feed filtrate separate from the first feed filtrate, the second feed filtrate comprising monocarboxylic acid solvent and solids, the second feed filtrate being lower in solids than the first feed filtrate; and
transferring at least a portion of the first feed filtrate to the reactor zone as recycle; and recovering oxidation catalyst from the second feed filtrate in a catalyst recovery zone.

In certain embodiments of the processes as otherwise described herein, oxidation catalyst is recovered from the second feed filtrate in a catalyst recovery zone.

In certain embodiments of the processes as otherwise described herein, at least a portion of the effluent of the catalyst recovery zone is recycled to the reactor zone.

In certain embodiments of the processes as otherwise described herein, the ratio of the volume of the first feed filtrate to the second feed filtrate is in the range of 1:20 to 3:1.

In certain embodiments of the processes as otherwise described herein, the ratio of the volume of the first feed filtrate to the second feed filtrate is in the range of 1:5 to 2:1.

In certain embodiments of the processes as otherwise described herein, the first feed filtrate includes at least 60% of the total amount of solids of all filtrates of the filtering operation.

In certain embodiments of the processes as otherwise described herein, the solid/liquid mixture is transferred to the rotary pressure filter from a crystallization zone in which the aromatic carboxylic acid is crystallized.

In certain embodiments of the processes as otherwise described herein, the filtering operation forms a filter cake, and wherein the process further comprises washing the filter cake in a first wash zone with a first wash fluid to form a first wash filtrate.

In certain embodiments of the processes as otherwise described herein, the ratio of the volume of the first wash filtrate to the first feed filtrate is in the range of 1:2 to 5:1.

In certain embodiments of the processes as otherwise described herein, at least a portion of the first wash filtrate is transferred to the reactor zone as recycle.

In certain embodiments of the processes as otherwise described herein, at least a portion of the first feed filtrate and, optionally, at least a portion of the first wash filtrate is transferred directly to the reactor zone as recycle.

In certain embodiments of the processes as otherwise described herein, at least a portion of the first feed filtrate and, optionally, at least a portion of the first wash filtrate is transferred indirectly to the reactor zone as recycle.

In certain embodiments of the processes as otherwise described herein, at least a portion of the first feed filtrate, and optionally, at least a portion of the first wash filtrate is transferred to a drum, and then at least a portion of the filtrate is transferred from the drum to the reactor zone.

In certain embodiments of the processes as otherwise described herein, the aromatic carboxylic acid comprises terephthalic acid.

In certain embodiments of the processes as otherwise described herein, the monocarboxylic acid solvent comprises acetic acid.

Other aspects of the disclosure will be apparent to those skilled in the art in view of the description that follows.

### BRIEF DESCRIPTION OF THE DRAWINGS

FIG. 1 is a process flow diagram for the manufacture and recovery of purified forms of aromatic carboxylic acids in accordance with one embodiment of the present disclosure.
FIG. 2 shows a side view cross-section of a rotary pressure filter in accordance with one embodiment of the present disclosure.
FIG. 3 shows a front view cross-section of a rotary pressure filter shown in FIG. 2.
FIG. 4 shows a perspective view of a portion of a rotary drum of the rotary pressure filter apparatus shown in FIGS. 2-3.
FIG. 5 shows a front view cross-section of a rotary pressure filter shown in FIGS. 2-4.

### DETAILED DESCRIPTION

In various aspects, the processes of the disclosure provide an efficient manner of recovering components of the filtrate of a solid/liquid mixture filtration.

Additional features of the processes of the disclosure will now be described in reference to the drawing figures.

The present inventors have noted that the filtrate collected from the filtration can vary as the material is processed through separate work zones, complicating the treatment and/or recovery of the components of the filtrate. Specifically in the context of separation of a solid crude aromatic carboxylic acid from a monocarboxylic acid solvent (e.g., formed by an oxidation reaction in the solvent), the present inventors have noted that the filter cake of the solid crude aromatic carboxylic acid itself can provide considerable filtering functionality. However, in the initial stage of filtration of the aromatic carboxylic acid from the solvent, the filter cake has not yet set, and so some solid aromatic carboxylic acid can be included with the filtrate. This is especially true when a filter with a relatively large pore size is used. Use of a relatively large pore size is advantageous, however, for purposes of increasing flow rate and simplification of rinsing.

The present inventors have determined that a rotary pressure filter can be used to separately collect filtrates from different filter zones, such that in an initial stage of filtration in a given zone (e.g., a feed zone) the higher-in-solids first feed filtrate is collected and recycled to a reactor zone, while in a subsequent stage of filtration the lower-in-solids (or even substantially free of solids) second feed filtrate can be conducted to a catalyst recovery zone, e.g., for removal of all or a part of an oxidation catalyst. Accordingly, one aspect of the disclosure provides a process including filtering a solid/liquid mixture having a solid crude aromatic carboxylic acid and a monocarboxylic acid solvent (e.g., from a crystallization zone) in a feed zone of a rotary pressure filter having at least two filter zones to form a first feed filtrate and then a second feed filtrate separate from the first feed filtrate, each of the first feed filtrate and the second feed filtrate comprising monocarboxylic acid solvent, the second feed filtrate being lower in solids than the first feed filtrate. By "lower in solids," it is meant that the second feed filtrate has a lower absolute amount of solids by mass than the first feed filtrate. In certain embodiments, the second feed filtrate has no more than 50%, no more than 25%, or even no more than 10% of the solids of the first feed filtrate as a mass percent. In this context, "solids" means material that is actually solid in the mixture (i.e., not dissolved solids).

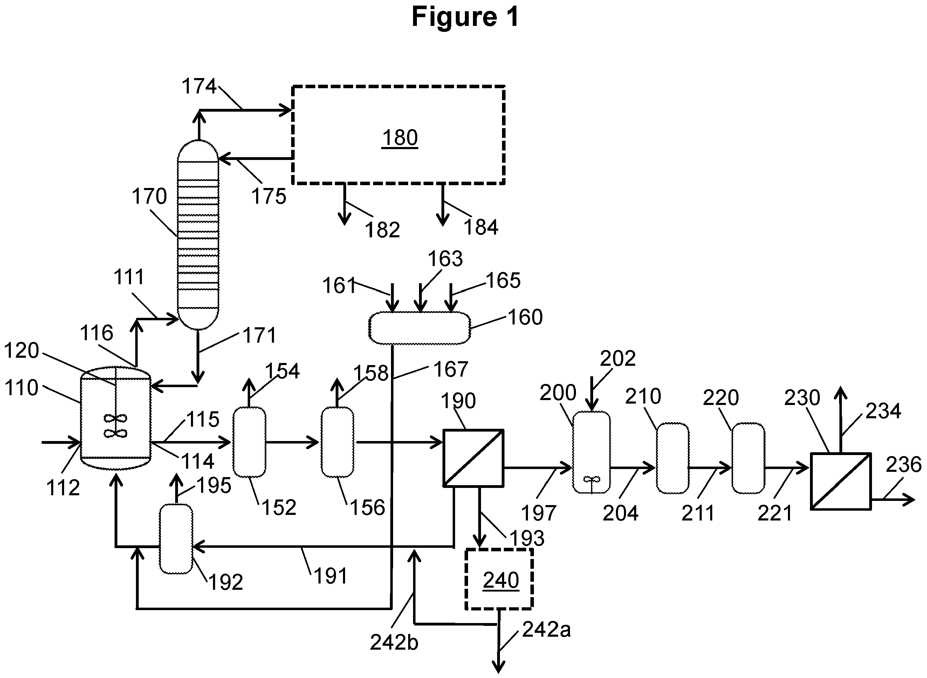

FIG. 1 is a process flow diagram for manufacturing and recovering aromatic carboxylic acids in accordance with one embodiment of the present disclosure. FIG. 1 depicts a number of additional elements for use in processes according to certain embodiments of the disclosure. A system for performing a process **100** of FIG. 1 includes a reaction zone that includes an oxidation reactor **110** configured for liquid-phase oxidation of feedstock to provide a reaction zone effluent; a crystallization zone **150** configured for forming solid crude aromatic carboxylic acid from the reaction zone effluent, and comprising crystallization vessels **152** and **156;** a solid/liquid separation device **190** configured for separating solid crude aromatic carboxylic acid (and oxidation by-products) from liquid; a mixing zone including a purification reaction mixture make-up vessel **200** configured for preparing mixtures of crude aromatic carboxylic acid in purification reaction solvent; a purification zone including a hydrogenation reactor **210** configured for contacting the crude aromatic carboxylic acid with hydrogen in the presence of a catalyst to form a purified aromatic carboxylic acid; a recovery zone comprising a crystallization zone **220** including at least one crystallizer configured for forming a slurry stream comprising solid purified aromatic carboxylic acid and a vapor stream, wherein the vapor stream comprises steam and hydrogen; and a solid/liquid separation device **230** configured for separating solid purified aromatic carboxylic acid from liquid.

However, the person of ordinary skill in the art will appreciate that the integration of processes in FIG. 1 is meant to be purely representative, and various other integrated and non-integrated configurations may likewise be used.

Liquid and gaseous streams and materials used in the process represented in FIG. 1 may be directed and transferred through suitable transfer lines, conduits, and piping constructed, for example, from materials appropriate for process use and safety. It will be understood that particular elements may be physically juxtaposed and, where appropriate, may have flexible regions, rigid regions, or a combination of both. In directing streams of compounds, intervening apparatuses and/or optional treatments may be included. By way of example, pumps, valves, manifolds, gas and liquid flow meters and distributors, sampling and sensing devices, and other equipment (e.g., for monitoring, controlling, adjusting, and/or diverting pressures, flows and other operating parameters) may be present.

As described above, the feedstock includes a substituted aromatic hydrocarbon. Representative feedstock materials suitable for use in the processes of the disclosure include but are not limited to aromatic hydrocarbons substituted at one or more positions with at least one substituent that is oxidizable to a carboxylic acid group. In some embodiments, the positions of the substituents correspond to the positions of the carboxylic acid groups of the aromatic carboxylic acid being prepared. In some embodiments, the oxidizable substituents include alkyl groups (e.g., methyl, ethyl, and/or isopropyl groups). In other embodiments, the oxidizable substituents include oxygen-containing groups, such as hydroxyalkyl, formyl, aldehyde, and/or keto groups. The substituents may be the same or different. The aromatic portion of feedstock compounds may be a benzene nucleus or it may be bi- or polycyclic (e.g., a naphthalene and/or anthracene nucleus). In some embodiments, the number of oxidizable substituents on the aromatic portion of the feedstock compound is equal to the number of sites available on the aromatic portion. In other embodiments, the number of oxidizable substituents on the aromatic portion of the feedstock is fewer than all such sites (e.g., in some embodiments 1 to 4 and, in some embodiments, 2). Representative feed compounds that may be used in accordance with the present teachings-alone or in combinations-include but are not limited to toluene; ethylbenzene and other alkyl-substituted benzenes; *o*-xylene; *p*-xylene; *m*-xylene; tolualdehydes, toluic acids, alkyl benzyl alcohols, 1-formyl-4-methylbenzene, 1-hydroxymethyl-4-methylbenzene; methylacetophenone; 1,2,4-trimethylbenzene; 1-formyl-2,4-dimethyl-benzene; 1,2,4,5-tetramethylbenzene; alkyl-, formyl-, acyl-, and hydroxylmethyl-substituted naphthalenes (e.g., 2,6-dimethylnaphthalene, 2,6-diethylnaphthalene, 2,7-dimethylnaphthalene, 2,7-diethylnaphthalene, 2-formyl-6-methylnaphthalene, 2-acyl-6-methylnaphthalene, 2-methyl-6-ethyl naphthalene, and the like); and the like; and partially oxidized derivatives of any of the foregoing; and combinations thereof. In some embodiments, the substituted aromatic compound comprises a methyl-, ethyl-, and/or isopropyl-substituted aromatic hydrocarbon. In some embodiments, the substituted aromatic compound comprises an alkyl-substituted benzene, *o*-xylene, *p*-xylene, *m*-xylene, or the like, or combinations thereof.

Aromatic carboxylic acids manufactured in accordance with the present disclosure are not restricted and include but are not limited to mono- and polycarboxylated species having one or more aromatic rings. In some embodiments, the aromatic carboxylic acids are manufactured by reaction of gaseous and liquid reactants in a liquid phase system. In some embodiments, the aromatic carboxylic acid comprises only one aromatic ring. In other embodiments, the aromatic carboxylic acid comprises a plurality (e.g., two or more) of aromatic rings that, in some embodiments, are fused (e.g., naphthalene, anthracene, etc.) and, in other embodiments, are not. In some embodiments, the aromatic carboxylic acid comprises only one carboxylic acid (e.g., -CO₂H) moiety or a salt thereof (e.g., -CO₂X, where X is a cationic species including but not limited to metal cations, ammonium ions, and the like). In other embodiments, the aromatic carboxylic acid comprises a plurality (e.g., two or more) of carboxylic acid moieties or salts thereof. Representative aromatic carboxylic acids include but are not limited to terephthalic acid, trimesic acid, trimellitic acid, phthalic acid, isophthalic acid, benzoic acid, naphthalene dicarboxylic acids, and the like, and combinations thereof. In some embodiments, the present teachings are directed to manufacture of pure forms of terephthalic acid including purified terephthalic acid (PTA) and so-called medium purity terephthalic acids.

A representative type of oxidation that may be performed in the oxidation zone (e.g., oxidation reactor **110**) is a liquid phase oxidation that comprises contacting oxygen gas and a feed material comprising an aromatic hydrocarbon having one or more substituents oxidizable to carboxylic acid groups in a liquid phase reaction mixture. In some embodiments, the liquid phase reaction mixture comprises a monocarboxylic acid solvent (e.g., acetic acid) and water in the presence of an oxidation catalyst comprising at least one heavy metal component (e.g., Co, Mn, V, Mo, Cr, Fe, Ni, Zi, Ce, Hf, or the like, and combinations thereof) and a promoter (e.g., halogen compounds, etc.). In some embodiments, the oxidation is conducted at elevated temperature and pressure effective to maintain a liquid phase reaction mixture and form a high temperature, high-pressure vapor phase. In some embodiments, oxidation of the aromatic feed material in the liquid phase oxidation produces aromatic carboxylic acid as well as reaction by-products, such as partial or intermediate oxidation products of the aromatic feed material and/or solvent by-products. In some embodiments, the aromatic carboxylic acid comprises terephthalic acid, and the oxidizing comprises contacting para-xylene with gaseous oxygen in a liquid phase oxidation reaction mixture that comprises acetic acid, water, and a bromine-promoted catalyst composition. The liquid-phase oxidation and associated processes may be conducted as a batch process, a continuous process, or a semi-continuous process. The oxidation may be conducted in the reaction zone, e.g., in one or more reactors.

In a representative embodiment, such as may be implemented as shown in FIG. 1, liquid feed material comprising at least about 99 wt.% substituted aromatic hydrocarbon, aqueous acetic acid solution (e.g., containing about 70 to about 95 wt.% acetic acid), soluble compounds of cobalt and manganese (e.g., such as their respective acetates) as sources of catalyst metals, bromine (e.g., hydrogen bromide) as catalyst promoter, and air, as a source of oxygen, may be continuously charged to oxidation reaction vessel **110** through inlets, such as inlet **112.** In some embodiments, vessel **110** is a pressure-rated, continuous-stirred tank reactor.

In some embodiments, stirring may be provided by rotation of an agitator **120,** the shaft of which is driven by an external power source (not shown). Impellers mounted on the shaft and located within the liquid body are configured to provide forces for mixing liquids and dispersing gases within the liquid body, thereby avoiding settling of solids in the lower regions of the liquid body.

In some embodiments, para-xylene is oxidized in reaction zone, predominantly to terephthalic acid. By-products that may form in addition to terephthalic acid include but are not limited to partial and intermediate oxidation products (e.g., 4-carboxybenzaldehyde, 1,4-hydroxymethyl benzoic acid, p-toluic acid, benzoic acid, and the like, and combinations thereof). Since the oxidation reaction is exothermic, heat generated by the reaction may cause boiling of the liquid phase reaction mixture and formation of an overhead gaseous stream that comprises vaporized monocarboxylic acid, water vapor, gaseous by-products from the oxidation reaction, carbon oxides, nitrogen from the air charged to the reaction, unreacted oxygen, and the like, and combinations thereof.

The gaseous stream may be removed from the reactor through vent **116** and sent in a stream **111** to a distillation column **170.** The distillation column **170** is configured to separate water from the solvent monocarboxylic acid and return a monocarboxylic acid-rich liquid phase to the reactor in stream **171.** A water-rich gaseous stream is removed from the distillation column **170** in stream **174** and sent for further processing to an off-gas treatment zone **180.** Reflux is returned to the distillation column **170** in stream **175.** The reflux liquid may include a condensed, liquid phase component **182** of the water-rich gaseous stream **174,** or may include fluid from other sources, such as liquid stream **234.**

The person of ordinary skill in the art will appreciate that the off-gas treatment zone can include a variety of components, for example, one or more of a condenser; a disengagement drum configured to separate the effluent of the condenser into a gas-phase component and a liquid-phase component; a scrubber configured to remove impurities (e.g., alkyl aromatic hydrocarbons, solvent monocarboxylic acid) from the gas-phase component; a catalytic oxidation ("catox") unit configured to remove impurities (e.g., organic components, HBr) from the gas-phase component; a bromine scrubber, configured to remove bromine from the gas-phase effluent of the catox unit; and an expander and a turbine, configured to convert energy from the gas-phase component to electricity. The components of the off-gas treatment zone may be arranged in a number of configurations. For example, the gas-phase effluent of the high-pressure bromine scrubber may be sent to the expander and turbine. In another example, the gas-phase effluent of the absorber may be sent to the expander and turbine. In yet another example, the gas-phase effluent of the disengagement drum may be sent to the expander and turbine. A liquid-phase component **182** is removed from the off-gas treatment zone **180,** and may include the liquid-phase effluent of the disengagement drum or the scrubber. A gas-phase component **184** is removed from the off-gas treatment zone **180,** and may include the gas-phase effluent of the disengagement drum, the absorber, the high-pressure bromine scrubber, or the expander and turbine.

The person of ordinary skill in the art will appreciate that the off-gas treatment zone can be configured in a variety of manners. Examples of processing and treatment of the reaction off-gas stream, and sources of reflux fluids are more fully described in U.S. Pat. Nos. 5,723,656, 6,137,001, 7,935,844, 7,935,845, and 8,173,834.

In some embodiments, solid crude product may be recovered from the reaction zone effluent by crystallization in one or more stages, such as in a single crystallization vessel, or, as shown in FIG. 1, in a series of multiple stirred crystallization vessels. In some embodiments, the crystallization process comprises sequential reductions in temperature and pressure from earlier to later stages to increase product recover. In the embodiment shown in FIG. 1, crystallization vessels **152** and **156** may be provided in series and in fluid communication, such that product slurry from vessel **152** may be transferred to vessel **156.** Cooling in the crystallizers may be accomplished by pressure release. One or more of the crystallizers may be vented, as at vents **154** and **158,** to remove vapor resulting from pressure let down and generation of steam from the flashed vapor to a heat exchanger (not shown).

In certain embodiments of the processes as otherwise described herein, the aromatic carboxylic acid prepared by the process comprises terephthalic acid, and the substituted aromatic hydrocarbon of the feedstock comprises para-xylene. For example, in certain such embodiments, the substituted aromatic hydrocarbon of the feedstock is at least 99% by weight para-xylene.

A variety of process operations can be used in recovery of the crystallized carboxylic acid. In certain embodiments as otherwise described herein, at least a portion of an effluent of a last crystallizer of the crystallization zone is separated to form an aromatic carboxylic acid-rich stream and a solvent-rich stream. For example, in the process depicted in FIG. 1, the crystallization vessel **156** is in fluid communication with a solid/liquid separation device **190.** The solid/liquid separation device **190** is configured to receive a slurry of solid product from the crystallization vessel **156,** and is further configured to separate the slurry of solid product to form an aromatic carboxylic acid-rich stream **197** and one or more solvent-rich streams (in FIG. 1, **191** and **193**). In a representative embodiment, such as may be implemented as shown in FIG. 1, the slurry of solid product from the crystallization vessel **156** includes a solid crude aromatic carboxylic acid, a monocarboxylic acid solvent, and minor amounts of an oxidation catalyst.

Notably, the separation methods described herein can advantageously be used in the separation of at least a portion of a last crystallizer of the crystallization zone to form an aromatic carboxylic acid rich stream and one or more solvent-rich streams. Accordingly, the solid/liquid separation device **190** can be a rotary filter such as a rotary pressure filter. Suitable rotary pressure filters are sold by BHS-Sonthofen and are disclosed for example, in U.S. Pat. Nos. 2,741,369, 7,807,060, U.S. Pat. App. 20050051473, US Pat. App. 20150182890, and WO 2016/014830.

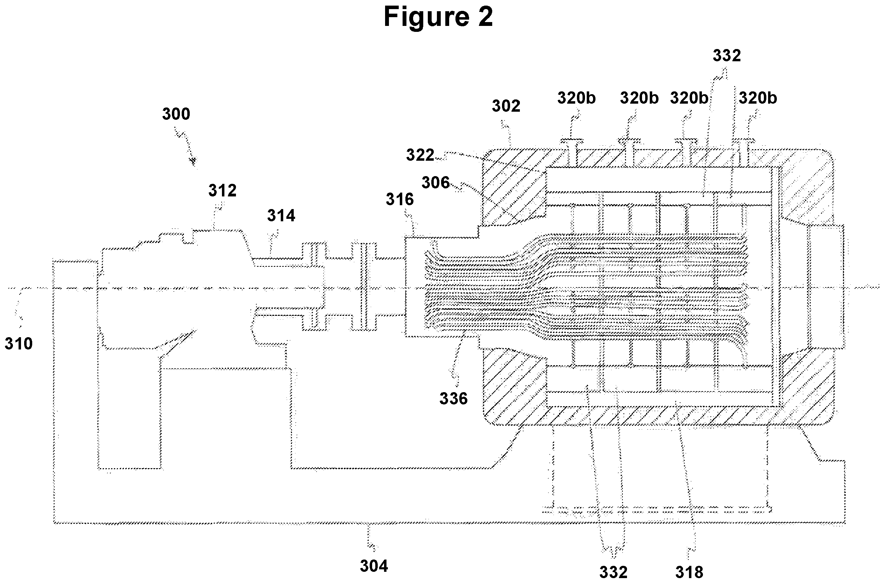

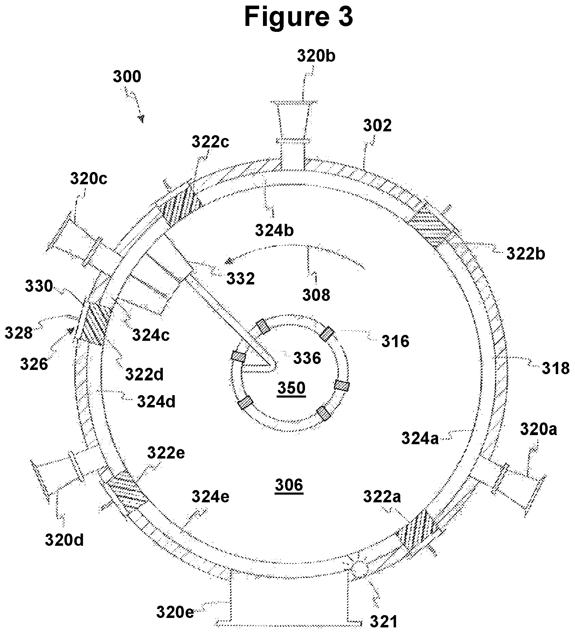

FIG. 2 is a longitudinal cross section of a rotary pressure filter apparatus in accordance with one embodiment of the present disclosure. FIG. 3 shows a front view cross-section of the rotary pressure filter shown in FIG. 2. As shown in FIG. 2, the rotary pressure filter apparatus **300** operates under a positive pressure to filter and remove liquid from a solid/liquid mixture and to collect a solid product as a final product or as an intermediate for further processing. The rotary pressure filter apparatus **300** includes a stationary housing **302** capable of withstanding an internal pressure above ambient. The housing **302** is mounted upon a frame **304.** Inside the housing **302** is a rotary filter drum **306.** As shown in FIG. 3, the rotary filter drum **306** rotates as indicated by arrow **308** around an axis **310** (FIG. 2) at a speed of around 0.4 to 2 RPM, and in some embodiments at a speed of about 0.8 to 1.5 RPM. The axis **310** defines a longitudinal direction of the rotary drum **306** and the rotary pressure filter apparatus **300.** The rotary filter drum **306** is driven by a drive mechanism **312,** which is also mounted on the frame **304.** A shaft **314** connects the drive mechanism **312** to a control head portion **316** of the rotary drum **306.**

The surface of the rotary drum **306** is spaced from the inside of the housing **302** such that generally annular plenum **318** is formed therebetween. Material passageways **320a, 320b, 320c, 320d,** and **320e,** such as inlets and outlet piping, are adapted to allow passage of material between the annular plenum **318** and a location outside the housing **302.**

One or more sealing members **322a, 322b, 322c, 322d, 322e** are configured to contact the rotary drum **306** and divide the annular plenum **318** into a plurality of zones **324a, 324b, 324c, 324d, 324e.** The sealing members **322** generally contact the rotary drum with enough pressure to pressure seal the zones **324** from each other but still allowing the rotary drum **306** to rotate. The sealing members **322** are each part of a sealing device **326** which includes an actuating mechanism adapted to members **322** in the radial direction to exert force against the rotary drum **306.** In the embodiment shown, the actuating mechanism is a pneumatic device including an inlet **328** for introducing gas into a plenum **330** to exert a pressure force against the outer surface of the respective sealing member **322.** Suitable pressure forces exerted by the pneumatic device include those about 0.8 to 2.0 bar above the highest pressure in any of the zones **324a-324e** of the rotary pressure filter apparatus **300.** Those skilled in the art will recognize that other actuating mechanisms may be substituted for the pneumatic device.

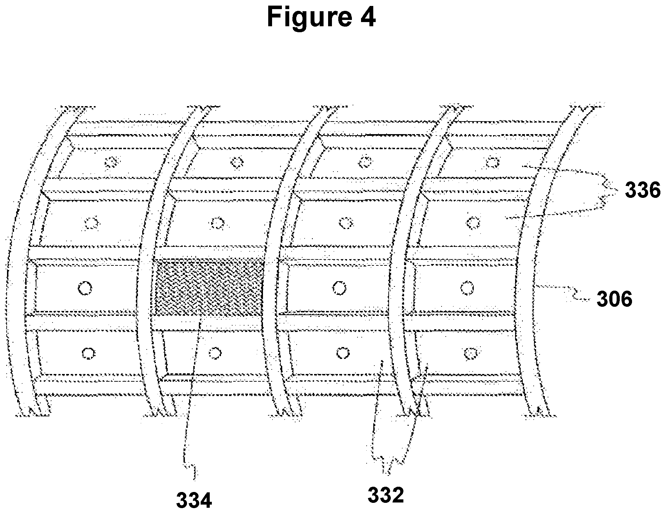

A plurality of compartments **332** are arranged around the outer surface or circumference of the rotary filter drum **306** and rotate with the filter drum **306.** The compartments **332** each include a filter member **334** (shown in one compartment in FIG. 4) adjacent the filter drum. In some embodiments the filter member comprises a filter sheet supported in a filter housing (not shown), for example, by being positioned over a metal screen. In some embodiments, the filter sheet is manufactured from a polyether ketone (PEEK) polymer or a polyvinylidene difluoride (PVDF) polymer. The filter sheet can be, for example, formed as a fabric or cloth.

The present inventors have noted that it can be especially desirable to use the methods described herein in conjunction with a filter member having a relatively large pore size. While relatively large pore sizes can be desirable from the standpoint of improved flow and resistance to fouling, they can allow particulate through in the initial stage of filtration, i.e., while the filter cake is forming. The processes described herein advantageously allow for the use of a filter member with a relatively large pore size, because the first feed filtrate (relatively rich in solids particles) can be recycled to the reaction zone, with the second feed filtrate (relatively poor in solids) being further processed, e.g., by going on to a catalyst recovery zone.

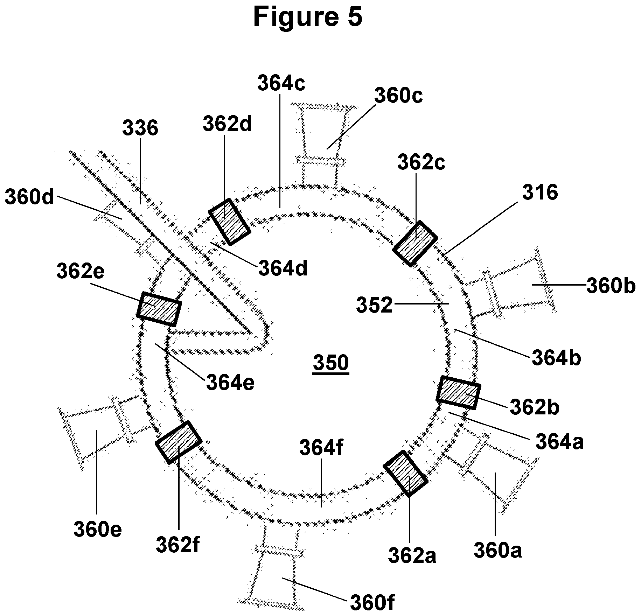

Each compartment **332** also has associated with it a corresponding outlet pipe **336** which also rotates with the filter drum **306** and the compartments **332.** The outlet pipes **336** are configured such that filtrate received from each compartment **332** passes through its corresponding filter member **334** adjacent the filter drum **306** and into its corresponding outlet pipe **336.** The outlet pipes **336** remove the filtrate from the compartments **332** and deliver the filtrate to the control head **316,** where it is collected through one or more filter zones **364** (shown in FIG. 5), and removed from the rotary pressure filter apparatus **300.**

The compartments **332** rotate with the rotary drum **306** and accordingly pass sequentially pass through each of the zones **324a, 324b, 324c, 324d, 324e.** In the embodiment shown, the compartments **332** are arranged in rows of four along the longitudinal direction **310.** Those skilled in the art will recognize that other configurations of the compartments would be suitable as well.

In operation, a pressurized feed containing a solid/liquid mixture is introduced into the feed inlet material passageway **320a** and into plenum **318** in a first zone designated as feed zone **324a.** The solid/liquid mixture is distributed into compartment **332.** In some embodiments, the pressure in the feed zone is maintained at about 3 bar(g) to about 7 bar(g), and in some embodiments, 5 bar(g) to 6 bar(g). As a result of the pressure differential that is maintained between the compartments **332** and the outlet pipes **336** and across the filter member **334** in the compartments, liquid and a portion of the solid components of the solid/liquid mixture is forced through the filter member **334** into outlet pipes **336.** Filtrate thus exits the rotary pressure apparatus **300** through outlet pipes **336** and is collected in a filter zone **350.** A portion of the solid components of the solid/liquid mixture remains on the filter members **334** in the form of a filter cake.

As the rotary drum **306** continues into the next zone **324b,** designated as a wash zone, wash fluid is introduced into plenum **318** for distribution into the compartments **332** to wash the cake remaining on the filter members **334.** In some embodiments, wash fluid is introduced at a rate of about 0.5 kg to about 1.5 kg of wash fluid per 1 kg of filter cake. The wash fluid is removed, as wash filtrate, by outlet **336.** In the embodiment shown, the rotary drum then continues to a second wash zone **324c,** where additional wash fluid is introduced into zone **324c,** designated as a second wash zone, and the cake on the filter members **334** is again washed.

The wash fluid is selected to remove impurities from the filter cake while not interfering with further processing of the filter cake to recover the final solid product. In one embodiment, the wash fluid comprises water. In another embodiment, the wash fluid comprises condensate from another portion of an integrated process.

The rotary drum 106 continues its rotation into drying zone **324d,** where a hot inert drying gas is introduced in the plenum **318** to dry the filter cake on the filter members **334.** As the rotary drum completes its rotation into discharge zone **324e,** the dried filter cake falls from the compartments **332** by gravity into a material passageway **320e** designated as a product chute (here, providing aromatic carboxylic acid-rich stream **197).** A rinse solution may be injected into inlet **321** in order to clean the filter members of the compartments **332** before they continue into the next cycle through the zones.

In the embodiment shown in FIGS. 2-4, the rotary pressure filter apparatus **300** includes a single feed zone **324a,** two wash zones **324b, 324c,** a single dry zone **324d,** and a single discharge zone **324e.** In other embodiments of the invention, the rotary pressure filter apparatus could have one wash zone, or more than two, or more or fewer than a single dry zone.

The circumference of the rotary pressure apparatus **100** defines a 360° work path, with each zone **324a, 324b, 324c, 324d,** and **324e** defining a portion of the work path.

FIG. 5 shows a cross-sectional view of control head **316** and inner housing **350.** Control head **316** is spaced from the inside of the inner housing **350** such that generally annular plenum **352** is formed therebetween. Material passageways **360a, 360b, 360c, 360d, 360e,** and **360f,** such as inlets and outlet piping, are adapted to allow passage of material between the annular plenum **352** and a location inside the inner housing **350.** One or more sealing members **362a, 362b, 362c, 362d, 362e,** and **362f** are configured to contact the control head **316** and divide the annular plenum **352** into a plurality of filter zones **364a, 364b, 364c, 364d, 364e,** and **364f.** In the embodiment shown in FIG. 5, filter zones **364c, 364d, 364e,** and **364f** each comprise a portion of the work path corresponding to, respectively, first wash zone **324b,** second wash zone **324c,** dry zone **324d,** and discharge zone **324e** of the rotary filter apparatus, and filter zones **364a** and **364b** together comprise a portion of the work path corresponding to the feed zone **324a** of the rotary pressure filter apparatus. The sealing members **362** generally contact the control head with enough pressure to pressure seal the filter zones **364** from each other but still allow the control head **316** to rotate.

Filtrate from compartments **332** is removed from the rotary pressure filter apparatus **300** through outlet pipes **336** and delivered into filter zones **364** that do not rotate with the filter drum **306.** In certain embodiments, filtrate from outlet pipes **336** is collected in six filter zones, **364a, 364b, 364c, 364d, 364e,** and **364f.**

The circumference of the rotary pressure apparatus **300** defines a 360° work path, with each filter zone **364** associated with a portion of the work path. In the embodiment shown in FIG. 5, filtrate from compartments **332** located in a first portion of feed zone **324a** will be delivered through outlet pipes **336** into filter zone **364a,** and removed from the rotary pressure filter apparatus **300** through outlet piping **360a,** and filtrate from compartments **332** located in a second portion of feed zone **324a** will be delivered through outlet pipes **336** into filter zone **364b,** and removed from the rotary pressure filter apparatus **300** through outlet piping **360b.** In certain embodiments, filter zone **364a** will collect filtrate from compartments **332** located in the first 40° of the feed zone **324a** of rotary pressure apparatus **300** (i.e., originating at sealing member **322a,** in rotation direction **308**), or in the first 60°, or in the first 80°, or in the first 100°, or in the first 120°, or in the first 140°, or in the first 160°, or in the first 180°, or in the first 200°, or in the first 220° of the feed zone **324a** of rotary pressure apparatus **300.**

In the embodiment shown in FIG. 5, a first wash filtrate from compartments **332** located in the first wash zone **324b** will be delivered through outlet pipes **336** into filter zone **364c,** and removed from the rotary pressure filter apparatus **300** through outlet piping **360c.**

In certain embodiments, the relative size and orientation of one or more filter zones **364** correspond to the size and orientation of one or more zones **324.** For example, in the embodiment shown in FIG. 5, the size and orientation of filter zone **364c** corresponds to that of first wash zone **324b,** and the size and orientation of filter zone **364d** corresponds to that of second wash zone **324c.** In other embodiments, the relative size and orientation of filter zones **364** is selected independently of the work path of the rotary pressure apparatus **300.** For example, in certain embodiments, filter zone **364a** will collect filtrate from compartments **332** of the first portion of the work path of rotary pressure apparatus **300** such that about 5 vol.% of the total filtrate delivered to the annular plenum **352** is collected in filter zone **364a,** or about 10 vol.%, or about 15 vol.%, or about 20 vol.%, or about 30 vol.%, or about 40 vol.%, or about 50 vol.%, or about 60 vol.% of the total filtrate delivered to the annular plenum **352** is collected in filter zone **364a.**

In certain embodiments, the ratio of the volume of the first feed filtrate (collected in filter zone **364a**) to the second feed filtrate (collected in filter zone **364b**) is in the range of 1:20 to 3:1, or 1:10 to 3:1, or 1:5 to 3:1, or 1:20 to 2.5:1, or 1:20 to 2:1, or 1:10 to 2.5:1, or 1:5 to 2:1. In the embodiment shown in FIG. 5, the first feed filtrate collected in filter zone **364a** and the second feed filtrate collected in filter zone **364b** each comprise monocarboxylic acid solvent. The person of ordinary skill in the art will appreciate that the filter cake of a compartment **332** can become more compressed as the compartment **332** moves in the rotation direction **308** (e.g., through zone **324a**). Accordingly, the amount of solids forced through the filter member **334** into outlet pipe **336** can decrease as the compartment **332** rotates with the rotary drum **306.** Notably, then, the presently disclosed processes can provide a second feed filtrate substantially lower in solids than the first feed filtrate.

In certain desirable embodiments, the first feed filtrate includes a high proportion of the total amount of solids collected in the filtrate streams of the filtering operation. For example, in certain embodiments, the first feed filtrate includes at least 60%, at least 75%, or even at least 90% of the total amount of solids of all of the filtrates of the filtering operation.

The first feed filtrate is transferred to a reaction zone as recycle. In certain embodiments, the first feed filtrate is indirectly transferred to oxidation reactor **110** as recycle. For example, in the embodiment shown in FIG. 5, the first feed filtrate is transferred through line **191** to mother liquor drum **192,** and a portion of the mother liquor is transferred from drum **192,** through line **193,** to oxidation reactor **110.** In another example, in the embodiment shown in FIG. 5, an overhead gaseous stream of the mother liquor drum **192** (i.e., comprising the first feed filtrate) can be condensed and transferred to solvent drum **160** (e.g., through line **161**) along with a condensed gaseous overhead stream of a crystallizer (e.g., from lines **154** and/or **158,** through line **163**) and/or make-up solvent (e.g., through line **165**). In such embodiments, a portion of the solvent is transferred from drum **160,** through line **167** and line **193,** to oxidation reactor **110.** In other embodiments, the first feed filtrate is directly transferred to a reaction zone.

In certain embodiments, the second feed filtrate is transferred through line **193** to a catalyst recovery zone **240.** Notably, the second filtrate feed, having a low solids content, can be especially suitable for catalyst recovery processes. In catalyst recovery zone **240,** all or a part of an oxidation catalyst can be removed from the second feed filtrate to provide a stream poor in catalyst **242.** A portion of the stream poor in catalyst **242a** is purged, and a portion of the stream poor in catalyst **242b** is transferred to a reaction zone (here, indirectly, with solvent-rich stream **191** to mother liquor drum **192,** and then to oxidation reactor **110**). In a representative embodiment, such as may be implemented as shown in FIG. 1, cobalt compounds are recovered from the second feed filtrate.

In certain embodiments, the ratio of the volume of the first wash filtrate (collected in filter zone **364c**) to the first feed filtrate (collected in filter zone **364a**) is in the range of 1:2 to 5:1, or 1:1 to 5:1, or 2:1 to 5:1, or 3:1 to 5:1, or 1:2 to 4:1, or 1:2 to 3:1, or 1:2 to 2:1, or 1:1 to 4:1, or 1:1 to 3:1. In certain embodiments, the first wash filtrate is transferred to a reaction zone as recycle. Notably, the first wash filtrate, having a low solids content but a relatively high water content, can desirably be directed to a zone other than the catalyst recovery zone, where one or more components of the wash filtrate could interfere with catalyst recovery processes. In certain embodiments, the first wash filtrate is combined with the first feed filtrate and transferred directly to a reaction zone. In certain embodiments, the first wash filtrate is combined with the first feed filtrate and transferred indirectly to a reaction. For example, in the embodiment shown in FIG. 5, the first wash filtrate and the first feed filtrate are combined and then transferred through line **191** to mother liquor drum **192,** and a portion of the mother liquor is transferred from drum **192,** through line **193,** to oxidation reactor **110.**

As shown in FIG. 1, the aromatic carboxylic acid-rich stream **197** from the separation device **190** comprising crude solid product may be directed to a mixing zone including a reaction mixture make up vessel **200.** The crude solid product in stream **197** may be mixed and slurried in make-up vessel **200** with a make-up solvent entering vessel **200** through line **202** to form a purification reaction mixture comprising crude aromatic carboxylic acid. The purification reaction mixture prepared in vessel **200** is withdrawn through line **204.** In some embodiments, the purification make-up solvent contains water. In some embodiments, the solvent line **202** connects to a holding vessel (not shown) for containing make-up solvent. In other embodiments, the solvent comprises fresh demineralized water fed from a deaerator. In other embodiments, the solvent is supplied from another part of the integrated process **100.** For example, in one embodiment, the solvent comprises liquid-phase component **182** of off-gas treatment zone **180.** In another embodiment, the solvent comprises the liquid-phase stream **234** exiting solid/liquid separator **230.** Sources of purification make-up solvent are more fully described, for example, in U.S. Pat. Nos. 5,723,656, 6,137,001, 7,935,844, 7,935,845, and 8,173,834.

In certain embodiments as otherwise described herein, at least a portion of the aromatic carboxylic acid-rich stream is purified in a purification zone comprising a hydrogenation catalyst under reaction conditions suitable to form a purification effluent comprising purified aromatic carboxylic acid. For example, in the process depicted in FIG. 1, purification reaction mixture exiting vessel **200** through stream **204** enters purification reactor **210** of the purification zone. The purification zone may further include a pump and one or more heat exchangers (not shown) configured to pre-heat the purification mixture exiting vessel **200** before it enters purification reactor **210.** In some embodiments, the purification reactor **210** is a hydrogenation reactor and purification in the purification reactor **210** comprises contacting the purification reaction mixture comprising crude aromatic carboxylic acid with hydrogen in the presence of a hydrogenation catalyst. In some embodiments, at least a portion of a purification effluent comprising purified aromatic carboxylic acid may be continuously removed from hydrogenation reactor **210** in stream **211** and directed to a crystallization zone **220** downstream of the purification zone. Crystallization zone **220** may comprise a plurality of crystallizers. In some embodiments, in crystallization zone **220,** purified aromatic carboxylic acid and reduced levels of impurities may be crystallized from the reaction mixture. The resulting solid/liquid mixture comprising purified carboxylic acid solids formed in crystallization zone **220** may be fed, in stream **221,** to a solid/liquid separation device **230,** configured to separate the solid/liquid mixture into a liquid-phase stream **234** and an aromatic carboxylic acid-rich stream **236** comprising solid purified aromatic carboxylic acid.

The foregoing detailed description and the accompanying drawings have been provided by way of explanation and illustration, and are not intended to limit the scope of the appended claims.

## Claims

1. A process for manufacturing an aromatic carboxylic acid comprising oxidizing in a reactor zone a feedstock comprising a substituted aromatic hydrocarbon in the
presence of an oxidation catalyst and monocarboxylic acid solvent under reaction conditions suitable to form crude aromatic carboxylic acid;
cooling the reactor zone effluent to form a solid/liquid mixture comprising a solid crude aromatic carboxylic acid, a monocarboxylic acid solvent, and minor amounts of the oxidation catalyst;
filtering the solid/liquid mixture in a feed zone of a rotary pressure filter, the feed zone having at least two filter zones to form
a first feed filtrate comprising monocarboxylic acid solvent and solids; and
a second feed filtrate separate from the first feed filtrate, the second feed filtrate comprising monocarboxylic acid solvent and solids, the second feed filtrate being lower in solids than the first feed filtrate;
transferring at least a portion of the first feed filtrate to the reactor zone as recycle; and recovering oxidation catalyst from the second feed filtrate in a catalyst recovery zone.

2. The process of claim 1, wherein at least a portion of the effluent of the catalyst recovery zone is recycled to the reactor zone.

3. The process of claim 1 or claim 2, wherein the ratio of the volume of the first feed filtrate to the second feed filtrate is in the range of 1:20 to 3:1.

4. The process of claim 1 or claim 2, wherein the ratio of the volume of the first feed filtrate to the second feed filtrate is in the range of 1:5 to 2:1.

5. The process of any of claim 1-4, wherein the first feed filtrate includes at least 60% of the total amount of solids of all of the filtrates of the filtering operation.

6. The process of any of claims 1-5, wherein the solid/liquid mixture is transferred to the rotary pressure filter from a crystallization zone in which the aromatic carboxylic acid is crystallized.

7. The process of any of claims 1-6, wherein the filtering operation forms a filter cake, and wherein the process further comprises washing the filter cake in a first wash zone with a first wash fluid to form a first wash filtrate.

8. The process of claim 7, wherein the ratio of the volume of the first wash filtrate to the first feed filtrate is in the range of 1:2 to 5:1.

9. The process of claim 7 or claim 8, further comprising transferring at least a portion of the first wash filtrate to the reactor zone as recycle.

10. The process of any of claims 1-9, comprising directly transferring at least a portion of the first feed filtrate and, optionally, at least a portion of the first wash filtrate to the reactor zone as recycle.

11. The process of any of claims 1-9, comprising indirectly transferring at least a portion of the first feed filtrate and, optionally, at least a portion of the first wash filtrate to the reactor zone as recycle.

12. The process of claim 11, comprising
transferring at least a portion of the first feed filtrate and, optionally, at least a portion of the first wash filtrate to a drum; and then
transferring at least a portion of the filtrate from the drum to the reactor zone.

13. The process of any of claims 1-12, wherein the aromatic carboxylic acid comprises terephthalic acid.

14. The process of any of claims 1-13, wherein the monocarboxylic acid solvent comprises acetic acid.

## Patentansprüche

1. Verfahren zum Herstellen einer aromatischen Carbonsäure, umfassend
Oxidieren eines Einsatzstoffes, der einen substituierten aromatischen Kohlenwasserstoff umfasst, in Gegenwart eines Oxidationskatalysators und eines Monocarbonsäurelösungsmittels in einer Reaktorzone unter Reaktionsbedingungen, die zur Bildung von roher aromatischer Carbonsäure geeignet sind;
Abkühlen des Reaktorzonenablaufs unter Bildung eines Feststoff/Flüssigkeits-Gemisches, das eine feste rohe aromatische Carbonsäure, ein Monocarbonsäurelösungsmittel und geringe Mengen des Oxidationskatalysators umfasst;
Filtrieren des Feststoff/Flüssigkeits-Gemisches in einer Zulaufzone eines Rotationsdruckfilters, wobei die Zulaufzone mindestens zwei Filterzonen aufweist zum Bilden von:
einem ersten Zulauffiltrat, umfassend Monocarbonsäurelösungsmittel und Feststoffe; und
einem vom ersten Zufuhrfiltrat getrennten zweiten Zufuhrfiltrat, wobei das zweite Zulauffiltrat Monocarbonsäurelösungsmittel und Feststoffe umfasst, wobei das zweite Zulauffiltrat in Feststoffen niedriger ist als das erste Zulauffiltrat;
Überführen zumindest eines Teils des ersten Zulauffiltrats in die Reaktorzone als Recycling; und
Rückgewinnen von Oxidationskatalysator aus dem zweiten Zulauffiltrat in einer Katalysatorrückgewinnungszone.

2. Verfahren nach Anspruch 1, **dadurch gekennzeichnet, dass** zumindest ein Teil des Abwassers der Katalysatorrückgewinnungszone in die Reaktorzone zurückgeführt wird.

3. Verfahren nach Anspruch 1 oder 2, **dadurch gekennzeichnet, dass** das Verhältnis des Volumens des ersten Zulauffiltrats zum zweiten Zulauffiltrat im Bereich von 1:20 bis 3:1 liegt.

4. Verfahren nach Anspruch 1 oder 2, **dadurch gekennzeichnet, dass** das Verhältnis des Volumens des ersten Zulauffiltrats zum zweiten Zulauffiltrat im Bereich von 1:5 bis 2:1 liegt.

5. Verfahren nach einem der Ansprüche 1 bis 4, wobei das erste Zulauffiltrat mindestens 60 % der Gesamtmenge an Feststoffen aller Filtrate des Filtervorgangs einschließt.

6. Verfahren nach einem der Ansprüche 1 bis 5, wobei das Feststoff/Flüssigkeits-Gemisch aus einer Kristallisationszone, in der die aromatische Carbonsäure kristallisiert wird, in den Rotationsdruckfilter überführt wird.

7. Verfahren nach einem der Ansprüche 1 bis 6, wobei der Filtervorgang einen Filterkuchen bildet, und wobei das Verfahren ferner das Waschen des Filterkuchens in einer ersten Waschzone mit einer ersten Waschflüssigkeit umfasst, um ein erstes Waschfiltrat zu bilden.

8. Verfahren nach Anspruch 7, wobei das Verhältnis des Volumens des ersten Waschfiltrats zum ersten Zulauffiltrat im Bereich von 1:2 bis 5:1 liegt.

9. Verfahren nach Anspruch 7 oder Anspruch 8, ferner umfassend das Überführen zumindest eines Abschnitts des ersten Waschfiltrats in die Reaktorzone als Rückführung.

10. Verfahren nach einem der Ansprüche 1 bis 9, umfassend das direkte Überführen zumindest eines Teils des ersten Zulauffiltrats und optional zumindest eines Teils des ersten Waschfiltrats in die Reaktorzone als Rückführung.

11. Verfahren nach einem der Ansprüche 1 bis 9, umfassend das indirekte Überführen zumindest eines Teils des ersten Einspeisefiltrats und optional zumindest eines Teils des ersten Waschfiltrats in die Reaktorzone als Rückführung.

12. Verfahren nach Anspruch 11, umfassend:
Überführen zumindest eines Teils des ersten Zulauffiltrats und gegebenenfalls zumindest eines Teils des ersten Waschfiltrats zu einer Trommel; und dann
Überführen zumindest eines Teils des Filtrats von der Trommel in die Reaktorzone.

13. Verfahren nach einem der Ansprüche 1 bis 12, wobei die aromatische Carbonsäure Terephthalsäure umfasst.

14. Verfahren nach einem der Ansprüche 1 bis 13, wobei das Monocarbonsäurelösungsmittel Essigsäure umfasst.

## Revendications

1. Procédé de fabrication d'un acide carboxylique aromatique comprenant
l'oxydation, dans une zone de réacteur, d'une matière première comprenant un hydrocarbure aromatique substitué en présence d'un catalyseur d'oxydation et d'un solvant acide monocarboxylique dans des conditions de réaction appropriées pour former de l'acide carboxylique aromatique brut ;
le refroidissement de l'effluent de la zone de réacteur pour former un mélange solide/liquide comprenant un acide carboxylique aromatique brut solide, un solvant acide monocarboxylique et des quantités mineures du catalyseur d'oxydation ;
le filtrage du mélange solide/liquide dans une zone d'alimentation d'un filtre sous pression rotatif, la zone d'alimentation présentant au moins deux zones de filtre pour former
un premier filtrat d'alimentation comprenant un solvant acide monocarboxylique et des solides ; et
un second filtrat d'alimentation séparé du premier filtrat d'alimentation, le second filtrat d'alimentation comprenant un solvant acide monocarboxylique et des solides, le second filtrat d'alimentation présentant une teneur plus faible en solides que le premier filtrat d'alimentation ;
le transfert d'au moins une partie du premier filtrat d'alimentation vers la zone du réacteur en tant qu'élément à recycler ; et
la récupération du catalyseur d'oxydation à partir du second filtrat d'alimentation dans une zone de récupération de catalyseur.

2. Procédé selon la revendication 1, dans lequel au moins une partie de l'effluent de la zone de récupération de catalyseur est recyclée vers la zone de réacteur.

3. Procédé selon la revendication 1 ou la revendication 2, dans lequel le rapport du volume du premier filtrat d'alimentation au second filtrat d'alimentation est dans la plage de 1:20 à 3:1.

4. Procédé selon la revendication 1 ou la revendication 2, dans lequel le rapport du volume du premier filtrat d'alimentation au second filtrat d'alimentation est dans la plage de 1:5 à 2:1.

5. Procédé selon l'une quelconque des revendications 1 à 4, dans lequel le premier filtrat d'alimentation comprend au moins 60 % de la quantité totale de solides de tous les filtrats de l'opération de filtration.

6. Procédé selon l'une quelconque des revendications 1 à 5, dans lequel le mélange solide/liquide est transféré vers le filtre sous pression rotatif à partir d'une zone de cristallisation dans laquelle l'acide carboxylique aromatique est cristallisé.

7. Procédé selon l'une quelconque des revendications 1 à 6, dans lequel l'opération de filtration forme un gâteau de filtration, et dans lequel le procédé comprend également le lavage du gâteau de filtration dans une première zone de lavage avec un premier fluide de lavage pour former un premier filtrat de lavage.

8. Procédé selon la revendication 7, dans lequel le rapport du volume du premier filtrat de lavage au premier filtrat d'alimentation est dans la plage de 1:2 à 5:1.

9. Procédé selon la revendication 7 ou la revendication 8, comprenant également le transfert d'au moins une partie du premier filtrat de lavage vers la zone de réacteur en tant qu'élément à recycler.

10. Procédé selon l'une quelconque des revendications 1 à 9, comprenant le transfert direct d'au moins une partie du premier filtrat d'alimentation et, éventuellement, d'au moins une partie du premier filtrat de lavage vers la zone de réacteur en tant qu'élément à recycler.

11. Procédé selon l'une quelconque des revendications 1 à 9, comprenant le transfert indirect d'au moins une partie du premier filtrat d'alimentation et, éventuellement, d'au moins une partie du premier filtrat de lavage vers la zone de réacteur en tant qu'élément à recycler.

12. Procédé selon la revendication 11, comprenant
le transfert d'au moins une partie du premier filtrat d'alimentation et, éventuellement, d'au moins d'une partie du premier filtrat de lavage dans un tambour ; et puis
le transfert d'au moins une partie du filtrat du tambour vers la zone de réacteur.

13. Procédé selon l'une quelconque des revendications 1 à 12, dans lequel l'acide carboxylique aromatique comprend l'acide téréphtalique.

14. Procédé selon l'une quelconque des revendications 1 à 13, dans lequel le solvant acide monocarboxylique comprend l'acide acétique.
